# EUROPEAN PATENT APPLICATION

(11) **EP 2 108 951 A1**
(43) Date of publication of application: **14.10.2009**
(21) Application number: 08103456.3
(22) Date of filing: 09.04.2008
(51) Int. Cl.: G01N 30/88

(54) **Method of obtaining BZM purity, quantity of [123I]IBZM labeled ligand and quantity of BZM free ligand**

(71) Applicant: Atomic Energy Council - Institute of Nuclear Energy Research, Lungtan, Taoyuan, Taiwan (TW)
(72) Inventor: Yang, Han-Hsing, 325, Taoyuan County (TW); Liu, Kung-Tien, 325, Taoyuan County (TW); Hsia, Yi-Chih, 325, Taoyuan County (TW); Su, Chang-Yung, 325, Taoyuan County (TW); Lin, Tai-Sheng, 325, Taoyuan County (TW); Yang, An-Shoei, 325, Taoyuan County (TW); Shen, Lie-Hang, 325, Taoyuan County (TW)
(74) Representative: Lang, Christian

(57) **Abstract**

A purity of BZM is analyzed by a high performance liquid Chromatography (HPLC) to know whether the purity is qualified. And a quantity of a labeled ligand ([¹²³I]IBZM) and a quantity of a free ligand (BZM) are analyzed by a liquid chromatograph tandem mass spectrometer (LC-MS/MS). The analysis result is used for maintaining a stable and qualified imaging agent. By maintaining the imaging agent, image interferences from background and receptor number are prevented.

## Description

### Field of the invention

The present invention relates to examining BZM ((S)-(-)-2-hydroxy-6-methoxy-N-[(1-ethyl-2-pyrrolidinyl)methyl]benzamide, molecular formula = C₁₅H₂₂N₂O₃, molecular weight = 278.35) solution, more particularly, relates to analyzing a purity of BZM and quantifications of labeled ligand of [^{1 2 3}I]IBZM ((S)-(-)-3-iodo-2-hydroxy-6-methoxy-N[(1-ethyl-2-pyrrolidinyl)methyl]benzamide, molecular formula = C₁₅H₂₁IN₂O₃ molecular weight = 400.24) and free ligand of BZM.

### Description of the Related Arts

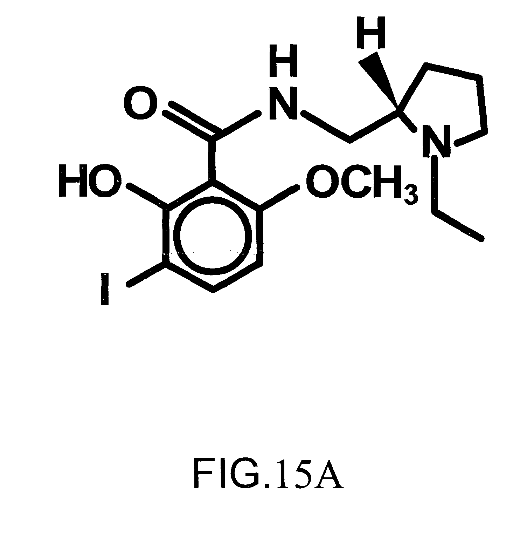

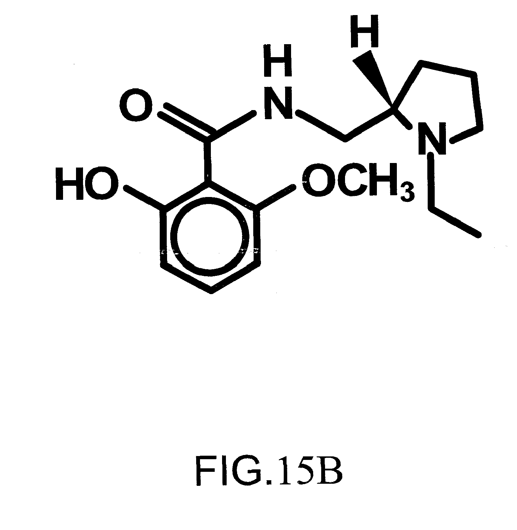

[^{12 3}I]IBZM, as shown in FIG.15A, is used as a striatal dopa minergic D2/D3 receptor (D2/D3R) single photon emission computed tomography (SPECT) imaging agent; and BZM, a free ligand as shown in FIG. 15B, is a precursor for [¹²³I]IBZM.

[¹²³I]IBZM is obtained through labeling BZM with I-123 (or ¹²³I). 135∼185 megabecquerels (MBq) of [¹²³I]IBZM is injected into a human body through an intravenous injection. Then, specific to nonspecific equilibrium ratios or coefficients in regions of interest (ROI) are obtained. Therein, for different cases, a specific bonding region can be striatum, caudate nucleus and putamen, or basal ganglia; and, a non-specific bonding region can be cerebellum, frontal cortex, or occipital cortex.

[¹²³I]IBZM SPECT imaging agent is widely applied in studies concerning striatal dopa minergic disease or D2 receptor occupancy, including studies on: Huntington's disease by Scherfler, etc. (Neurolmage, 2005, Vol.24, pp.822-831); on dopa mine release following ad ministration of ampheta mine by Abi-Dargham, etc. (Biol. Psychiatry, 2004, Vol.55, pp. 1001-1006; European Neuropsychopharmacology, 2003, Vol.13, pp.459-468); on obsessive-compulsive disorder (OCD) by Denys, etc. (Biol. Psychiatry, 2004, Vol.55, pp.1041-1045); on cognitive performance and fine motor activity by Yang, etc. (Psychiatry Research: Neuroimaging, 2004, Vol.131, pp.209-216; Psychiatry Research: Neuroimaging, 2003, Vol.123, pp.191-197); on Cotard syndrome De Risio, etc. by (Psychiatry Research: Neuroimaging, 2004, Vol.130, pp.109-112); on prognosis in naive schizophrenic patients by Pérez, etc. (Progress in Neuro-Psychopharmacology & Biological Psychiatry, 2003, Vol.27, pp.767-770); and on differences between nonidiopathic parkinsonism (NIPS) disorders and Parkinson's Disease (PD) by Radau, etc. (J. Nucl. Med., 2000, Vol.41, pp.220-227).

Besides, [¹²³I]IBZM SPECT imaging agent is used for evaluating clinical curative effects of antipsychotic agents (e.g. olanzapine, clozapine, haloperidol, sertiridole, risperidone, quetiapine, etc.) by Frankle, etc. (Psychopharmacology 2004, Vol.175, pp.473-480); Tauscher etc. (Psychopharmacology, 1999, Vol.141, pp.175-181); Kasper, etc. (Psychopharmacology, 1998, Vol.136, pp.367-373); and Küfferle, etc. (Psychopharmacology, 1997, Vol.133, pp.323-328). (¹²³I]IBZM is also used on studying extrapyramidal side effects (EPS) and acute psychosis exacerbation by Corripio, etc. (Progress in Neuro-Psychopharmacology & Biological Psychiatry, 2005, Vol.29, pp.91-96) and Heinz, etc. (Schizophrenia Research, 1998, Vol.31, pp.19-26).

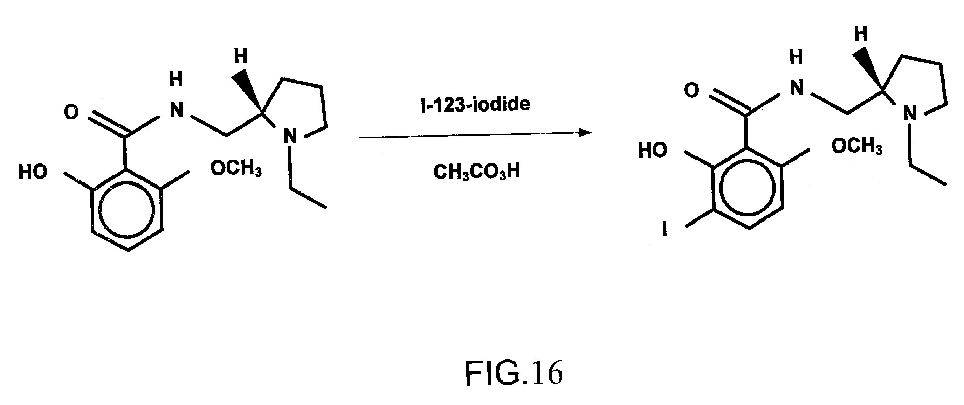

As shown in FIG.16, a labeling method of [¹²³I]IBZM is developed by Kung, etc. (Nucl. Med. Biol., 1988, Vol.15, pp.195-201; J. Med. Chem., 1988, Vol.31, pp.1039-1043; J. Labeled Compd. Radiopharm., 1988, Vol.27, pp.691-700; Nucl. Med. Biol., 1988, Vol.15, pp.203-208), Zea-Ponce, etc. (Nucl. Med. Biol., 1999, Vol.26, pp.661-665; Nucl. Med. Biol., 1999, Vol.26, pp.811-814) and Baldwin, etc. (Bioorganic & Medicinal Chemistry Letters, 2003, Vol.13, pp.4015-4017). [¹²³I]IBZM can be separated and purified from free ligand of I-123, BZM and the other impurities through high performance liquid chromatography (HPLC). However, eluent for HPLC contains acetonitrile or methanol and thus is not fit to be injected into human body. Hence, purified product collected through HPLC is added with reductant (e.g. ascorbic acid) to be dried through evaporation; and then is dissolved into ethanol or/and normal saline, where the whole process takes three hours (Bioorganic & Medicinal Chemistry Letters, 2003, Vol.13, pp.4015-4017 by Baldwin, etc.; J. Nucl. Med., 1990, Vol.31, pp.573-579 by Kung, etc.; Nucl. Med. Biol., 1999, Vol.26, pp.661-665 by Zea-Ponce, etc.; Nucl. Med. Biol., 1999, Vol.26, pp.811-814 by Zea-Ponce, etc.; Nucl. Med. Biol., 1988, Vol.15, pp.195-201 by Kung, etc.; J. Labeled Compd. Radiopharm., 1988, Vol.27, pp.691-700 by Kung, etc.)

Solid phase extraction (SPE) is therefore developed by Leslie, etc. (J. Nucl. Med., 1996, Vol.37, 1589-1591) because I-123 has a short hawf-life (T_{1/2} = 13.2 hrs, gamma energy = 159keV); and, complex processes for samples increase medicine contamination, radioactive material contamination and chemical decomposition and reduce [¹²³I]IBZM activity. Reactants, products and byproducts are all adhered on a SPE device to remove unreacted I-123 ions through being washed by normal saline. Then (¹²³I]IBZM is eluted by 95% ethanol to be diluted with normal saline for obtaining an injection. This method fast obtains a condensed product and processes labeling automatically for reducing radiation dosage on human body. However, [¹²³I]IBZM is not able to be separated from BZM precursor.

Quality control of [¹²³I]IBZM nuclear medicine is done through analyzing a radiochemical purity (RCP) for now. And a method for analyzing a RCP of an injection through thin-layer chromatography (TLC) and HPLC is developed by Kung, etc. (J. Labeled Compd. Radiopharm., 1988, Vol.27, pp.691-700), where the RCP for analyzing the purity of [¹²³I]IBZM is limited to be not smaller than 95% (Neurolmage, 2005, Vol.24, pp.822-831 by Scherfler, etc.; Biol. Psychiatry, 2004, Vol.55, pp.1001-1006 by Abi-Dargham, etc.; Psychopharmacology 2004, Vol.175, pp.473-480 by Frankle, etc.; Biol. Psychiatry, 2004, Vol.55, pp.1041-1045 by Denys, etc.; Schizophrenia Research, 1998, Vol.31, pp.19-26 by Heinz, etc.; J. Nucl. Med., 1990, Vol.31, pp.573-579 by Kung, etc.; J. Nucl. Med., 1989, Vol.30, pp.88-92 by Kung, etc.; Nucl. Med. Biol., 1999, Vol.26, pp. 661-665 by Zea-Ponce, etc.; Nucl. Med. Biol., 1988, Vol.15, pp.195-201 by Kung, etc.; J. Med. Chem., 1988, Vol.31, pp.1039-1043, by Kung, etc.; Nucl. Med. Biol., 1988, Vol.15, pp.203-208 by Kung, etc.)

However, only bonded or free ligand (ionic) I-123 activity can be detected by RCP, rather than concentration of nonradioactive materials such as BZM or other impurities. The injection may contain only a tiny sum of BZM or I-123; and Kung, etc. conclude in their study that BZM has a far small bonding to D2/D3 receptor (D2/D3R) than [¹²³I]IBZM in rats (J. Nucl. Med., 1989, Vol.30, pp.88-92). But, until now, no clinic test has proven that bonding of BZM and [¹²³I]IBZM to D2/D3R in human body is acted in the same way as in rats. It is quite possible that the competition between BZM and [¹²³I]IBZM bonding to D2/D3R in human body may affect [¹²³I]IBZM SPECT images such as unexpectedly interference, increased background or decreased detectable receptors.

As above, although a few methods for purifying [¹²³I]IBZM through HPLC are developed, no validated method is developed for analyzing purity of labeled BZM; similarly, no effective method is developed for fast analyzing quantification of labeled precursor or free ligand of BZM in the [¹²³I]IBZM injection.

### Summary of the invention

The main purpose of the present invention is to analyzing the purity of free ligand BZM, or of the precursor which used to label as a striatal dopa minergic D2/D3 receptor SPECT imaging agent, for qualifying the purity.

Another purpose is to obtain quantities of labeled ligand of [¹²³I]IBZM and free ligand of BZM in the striatal dopa minergic D2/D3 receptor SPECT imaging agent, especially those which could interfere with SPECT images, increase background and decrease number of detectable receptors, by multiple reaction monitoring (MRM) of liquid chromatograph tandem mass spectrometer (LC-MS/MS) for maintaining a stable and qualified intravenous SPECT imaging injection agent.

To achieve the above purposes, the present invention is a method of obtaining a BZM purity, a quantity of [¹²³I]IBZM labeled ligand and a quantity of BZM free ligand, where HPLC is used to analyze a purity of BZM for its specificity, linearity, accuracy, etc.; and an LC-MS/MS having an electrospray ionization (ESI) scan mode and a positive ion scan mode is provided to examine quantities of labeled ligand of [¹²³I]IBZM and free ligand of BZM though MRM for obtaining linearity of MRM transitions for the quantification of nonradioactive IBZM and [¹²³I]IBZM with fragmentation pathways of parent molecules of BZM, nonradioactive IBZM and [¹²³I]IBZM.

### Brief description of the drawings

The present invention will be better understood from the following detailed description of the preferred embodiment according to the present invention, taken in conjunction with the accompanying drawings, in which
- FIG.1: is the view showing the HPLC chromatogram of the BZM purity;
- FIG.2: is the view showing the HPLC chromatogram of the BZM forced degradation;
- FIG.3: is the view showing the Q1 mass spectra of BZM;
- FIG.4: is the view showing the Q1 mass spectra of nonradioactive IBZM;
- FIG.5: is the view showing the fragmentation pathways of the BZM parent molecule;
- FIG.6: is the view showing the fragmentation pathways of the nonradioactive IBZM parent molecule;
- FIG.7: is the view showing the fragmentation pathways of the [¹²³I]IBZM parent molecule;
- FIG.8: is the view showing the intra- day precision of the BZM purity analysis through HPLC;
- FIG.9: is the view showing the inter- day precision of the BZM purity analysis through HPLC;
- FIG.10: is the view showing the accuracy of the BZM purity analysis through HPLC;
- FIG.11: is the view showing the robustness of the BZM purity analysis through HPLC;
- FIG.12: is the view showing the solution stability of the BZM solution;
- FIG.13: is the view showing the best LC-MS/MS analysis parameters of IBZM and BZM;
- FIG.14: is the view showing the MRM analysis of IBZM and BZM;
- FIG.15A and FIG.15B: are the chemical- structure views of IBZM and BZM; and
- FIG.16: is the view of a general [¹²³I]IBZM labeling method.

### Description of the preferred embodiment

The following description of the preferred embodiment is provided to understand the features and the structures of the present invention.

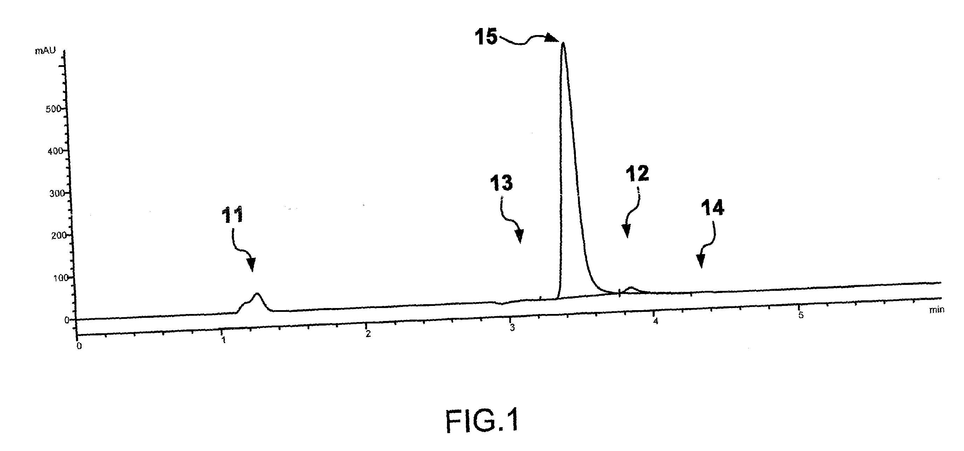

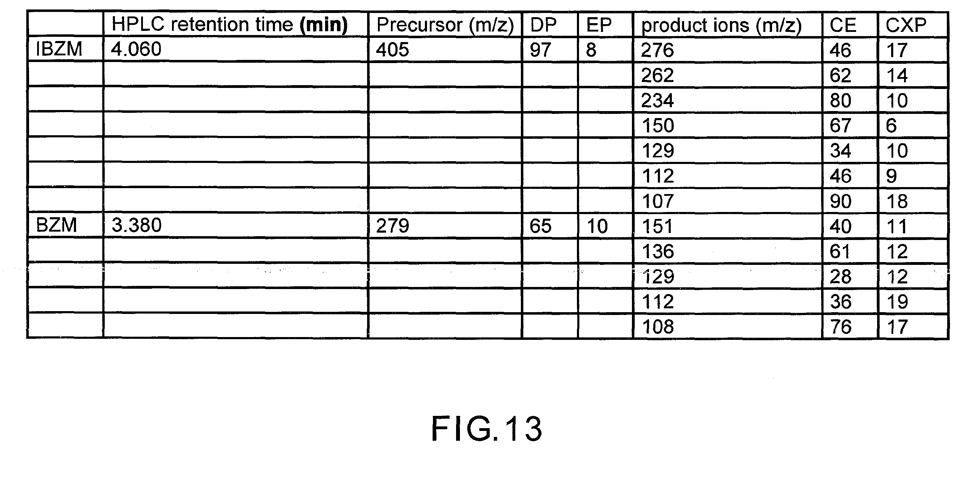

Please refer to FIG.1 to FIG.14, which are views showing an HPLC chromatogram of a BZM purity and an HPLC chromatogram of a BZM forced degradation; views showing Q1 mass spectra of BZM and IBZM; views showing fragmentation pathways of a BZM parent molecule, a nonradioactive IBZM parent molecule and a [¹²³I)IBZM parent molecule; views showing an intra-day precision and an inter-day precision of a BZM purity analysis through HPLC; a view showing an accuracy and a robustness of the BZM purity analysis through HPLC; a view showing a solution stability of a BZM solution; a view showing best LC-MS/MS analysis parameters of IBZM and BZM; and a view showing an MRM analysis of IBZM and BZM. As shown in the figures, facilities and medicines required for the present invention includes:
(1) a high performance liquid chromatography (HPLC) device, comprising a thermostated column oven and an ultra-violet detector;
(2) a liquid chromatograph tandem mass spectrometer (LC-MS/MS) having an electrospray ionization (ESI);
(3) a Zorbox Eclipse XDB-C18 column, 4.6 x 50 mm, 1.8 µm (Agilent, USA);
(4) dimethyl sulfoxide (DMSO) above analytical grade;
(5) ammonium acetate above analytical grade; and
(6) acetonitrile above analytical grade or chromatographic grade.

Solutions are prepared for the present invention as follows:
(1) BZM or IBZM samples are dissolved in a DMSO solution. Thus, a -500 ppm BZM or IBZM solution is provided to be examined through HPLC.
(2) BZM samples and IBZM samples are separately dissolved in the DMSO solutions; and then the DMSO solutions are diluted with acetonitrile to a required calibration curve range to be mixed together. Thus, a mixture solution of 2-45 ppm BZM and -500 ppm IBZM is obtained to be examined through HPLC.
(3) DMSO solutions having -500 ppm BZM and IBZM separately are obtained to be diluted with acetonitrile to a required concentration range. Thus, -100 ppb BZM and IBZM solutions are obtained to be processed through multiple reaction monitoring (MRM) by the LC-MS/MS.
(4) A mixture solution of 2-45 ppm BZM and -500 ppm IBZM is obtained to be diluted with acetonitrile to obtain a 5000 times of solution having a required concentration range. Thus, a mixture solution of 0.4∼10 ppb BZM and -100 ppb IBZM is obtained to be processed through MRM by the LC-MS/MS.

The present invention utilizes HPLC for analyzing the BZM purity; and, on obtaining best conditions for the present invention, nonradioactive IBZM ([¹²⁷I]IBZM) is used to replace radioactive IBZM ([¹²³I]IBZM). Although [¹²⁷I]IBZM and [¹²³I]IBZM have different molecular weights (mw 404 and mw 400), their chemical characteristics are similar, such as HPLC retention time. Hence, the best HPLC chromatographic conditions for the nonradioactive IBZM ([¹²⁷I]IBZM) can be applied to the radioactive IBZM ([¹²³I]IBZM).

The BZM purity is analyzed through the following steps:
(a) An HPLC device is obtained.
(b) The HPLC device is used to examine [¹²³I]IBZM and BZM for obtaining their specificity, linearity, accuracy, precision, limit of detection (LOD), limit of quantification (LOQ), robustness, and solution stability.

Concerning essence represented at HPLC chromatographic peaks, a mass spectrometer is used with a Q1 scan to obtain product ion mass of parent molecule. Then the mass spectrometer uses a precursor ion scan and a product ion scan to obtain structures of fragments for affirming the essences of BZM and IBZM. The eluent for HPLC is a mixture solution of a 10 mM pH 7.0 ammonium acetate buffer and acetonitrile to avoid precipitating, crystallizing and ion suppressing. Hence, the mixture solution can be directly injected into the mass spectrometer without changing components of the eluent or reducing its salt concentration

Therein, chromatographic conditions for analyzing the BZM purity include:
(1) a first eluent, a 10 mM pH 7.0 ammonium acetate buffer;
(2) a second eluent, acetonitrile;
(3) a gradient condition of eluting, comprising 10%-95% B (0.5 min), 95% B (0.5-6.0 min) and 10% B (6.0∼10.0 min);
(4) a temperature at 25°C for a column;
(5) a flow rate of 0.5 mL/min; and
(6) a detection wavelength at 254 nm.

As shown in FIG.1, peaks around retention times (t_{R}) of 1.241 min, 3.852 min, 3.105 min and 4.378 min are a solvent peak 11, a peak of main impurity 12 and peaks of minor impurities 13,14. Peak around retention time of 3.452 min is processed through a Q1 scan in a positive ion mode to affirm a protonated molecular ion ([M+H]⁺) of BZM at mass-to-charge ratio (m/z) of 279. In the same way, a protonated molecular ion of nonradioactive IBZM at m/z 405 is affirmed. Concerning HPLC chromatogram, main impurity can be found, yet minor impurities are not always found; and, average quantity is as low as 0.01∼0.02%.

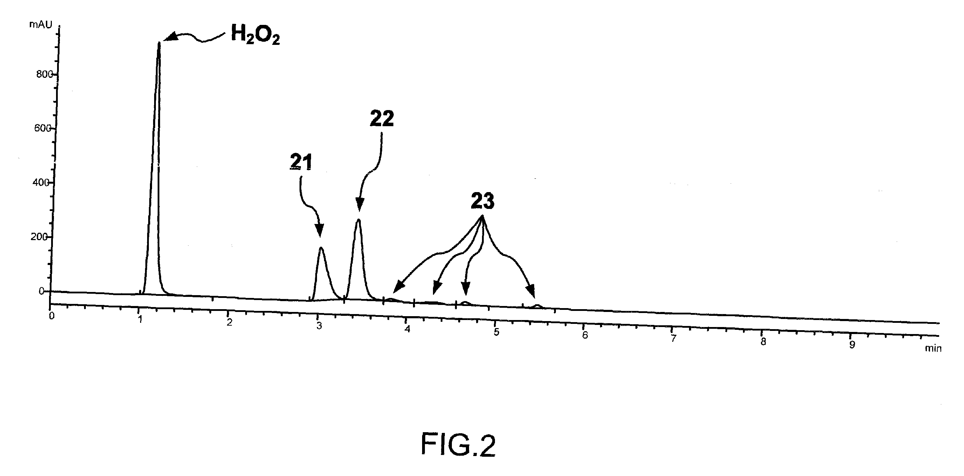

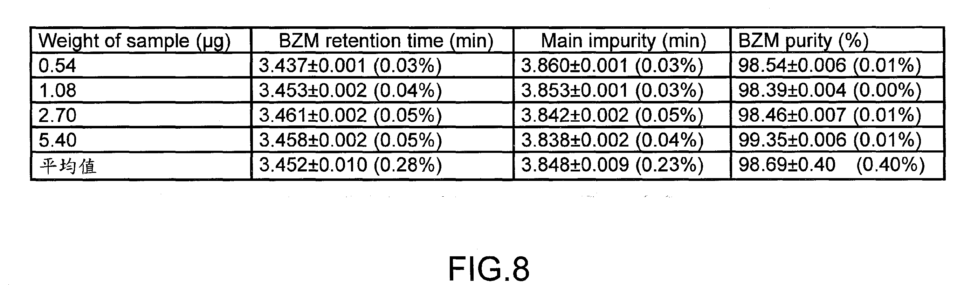

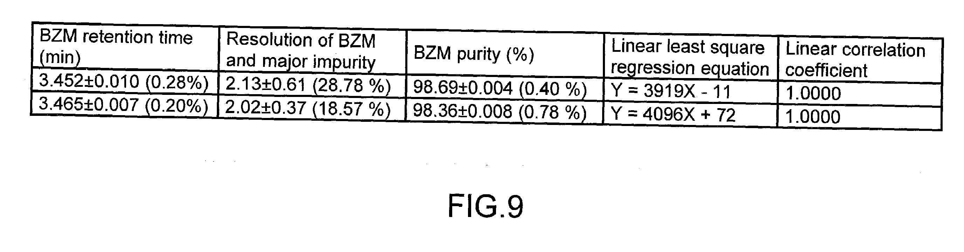

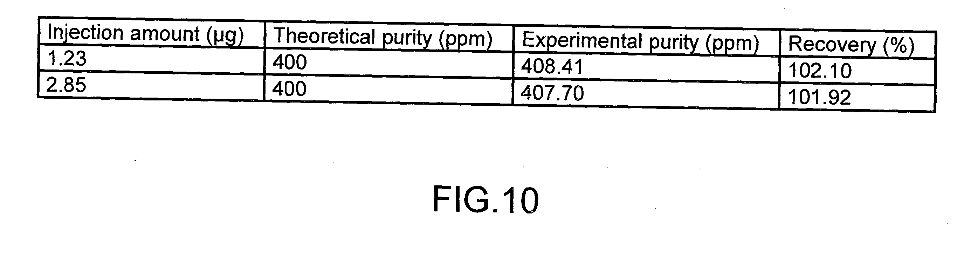

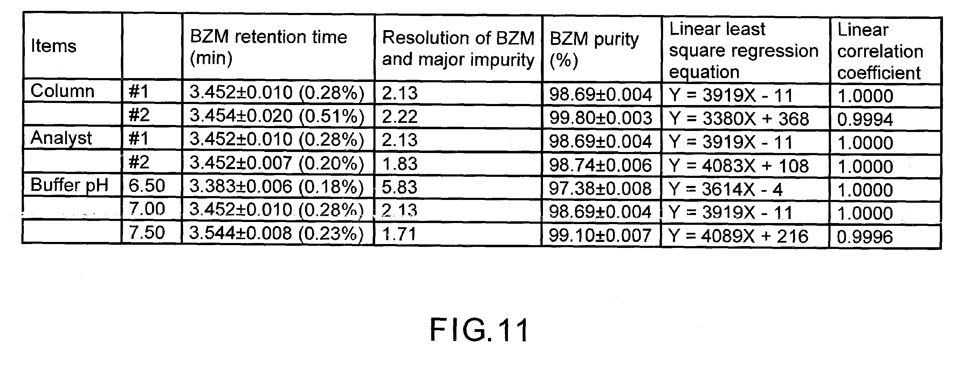

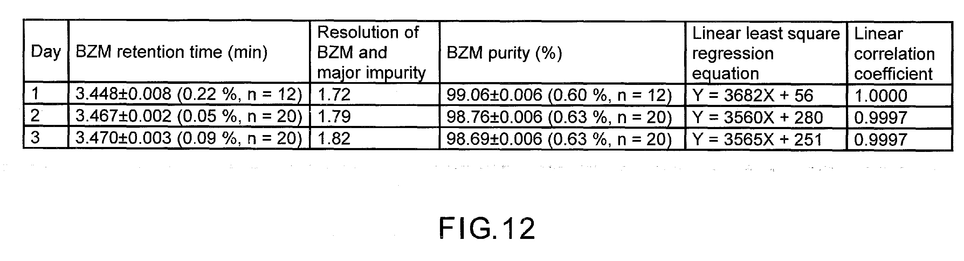

According to guidance documented by International Conference on Harmonization (ICH), results for confirming the method of analyzing the BZM purity include specificity, linearity, accuracy, precision, LOD, LOQ, robustness and solution stability, as shown in the followings:
(1) On specificity test, a forced degradation is used. 0.63 mg BZM is dissolved in 1.2 mL 3% H₂O₂ aqueous solution (oxidant) to react for 24 hours. As shown in FIG.2, a resolution of BZM 22 and main forced degradation compound 21 (t_{R} = 3.016 min) is 1.88, which shows that the present invention has a good specificity. BZM 22 is totally separated from the main forced degradation compound 21 and minor forced degradation compound 23 without affecting the quantification.
(2) For linearity test, a DMSO solution having 500 ppm BZM is obtained at first. 1∼10 µL sample is injected for five times. Then all chromatographic peaks obtained through HPLC are calculated for obtaining an average of peak areas, a standard deviation (SD) of peak areas and a relative standard deviation (RSD) of peak areas. Then, weights (microgram, µg of the samples are used for chromatographic peaks to obtain an average of peak areas (milliabsorbance unit, mAU) for figuring out linear least square regression equations and linear correlation coefficients. As shown in FIG.8, a linearity working range is 0.54∼5.4 µg; the linear least square regression equation is Y = 3919X-11; and the linear correlation coefficient is 1.0000. Consequently, the method of analyzing the BZM purity is shown to have a proper working range and a good linearity.
(3) Precision tests comprise an intra-day precision (i.e. repeatability) test and an inter-day precision (i.e. reproducibility) test:
   (31 ) A result of the intra-day precision test is shown in FIG.8, where a linearity working range is 0.54∼5.4 µg; a linear least square regression equation is Y = 3919X-11; and a linear correlation coefficient is 1.0000; a BZM purity is 98.69 ± 0.40 (RSD = 0.40%, n = 20); an average resolution of BZM and the main impurity 12 (t_{R} = 3.852 min) is 2.13 ± 0.61 (RSD = 28.78%, n = 20); and average theoretical plate numbers of BZM and the main impurity 12 are 4086 and 21134. Consequently, it is shown that the present invention has a good repeatability.
   (32) A result of the inter-day precision test is shown in FIG.9, where BZM has a retention time difference of 0.38%, a resolution difference of -5.16%, a purity difference of -0.33%, a difference of a linear least square regression slope of 4.52%, and a linear correlation coefficient of 1.0000. Consequently, it is shown that the present invention has a good reproducibility.
(4) The accuracy test is done through a recovery test. A BZM having a nominal concentration is spiked in DMSO to be analyzed by HPLC. Experimental concentration is obtained through Interpolation and recovery rate is calculated (= experimental concentration / nominal concentration x 100%). As shown in FIG.10, average recovery rates are 102.10% and 101.92%
(5) For LOD/LOQ test, three-times noise (signal-to-noise (S/N) ratio = 3/1) and ten-times noise (S/N ratio = 10/1) are used for calculation. The main impurity 12 is shown around 3.852 min. Yet, the minor impurities 13, 14 (in FIG.1, shown around 3.105 min and 4.378 min) are shown only when there is a high concentration or a lot of samples. Besides, average quantities of the minor impurities 13, 14 are as low as around 0.01∼0.02%. Hence, LOD/LOQ test are mainly on the main impurity 12. Moreover, 0.54 µg of sample is injected to be analyzed by HPLC. Thus, average nominal concentrations of BZM and the main impurity 12 is obtained as 8938 ± 949 (RSD = 10.62%, n = 4) and 158 ± 17 (RSD = 10.65%, n = 4). The LOD and the LOQ (quantity percentage) of BZM are 0.183 ± 0.021 µg (RSD = 11.23%, n = 4) and 0.610 ± 0.068 pg (RSD = 11.23%, n = 4) respectively. And, the LOD and the LOQ (quantity percentage) of the main impurity 12 are 0.028 ± 0.003% (RSD = 11.14%, n = 4) and 0.094 ± 0.010% (RSD = 11.14%, n = 4) respectively.
(6) The robustness test uses different HPLC columns (the same brand and type but different batch), different analyzers, and the same ammonium acetate buffer (the first eluent) with different pH values (pH 6.5, 7.0 and 7.5), as shown in FIG.11:
   (61) Concerning the results with different HPLC columns, the difference of BZM retention time is 0.06%; the resolution difference is 4.23%; the purity difference is 1.12%; the difference of a linear least square regression slope is - 13.75%; and linear correlation coefficients are all grater than 0.9994.
   (62) Concerning the results with different analyzers, the difference of BZM retention time is 0.00%; the resolution difference is -14.08%; the purity difference is 0.05%; the difference of a linear least square regression slope is 4.18%; and linear correlation coefficients are all grater than 0.9999.
   (63) Concerning the results with different pH values, difference of BZM retention time is reduced following reducing the pH value. A difference of resolution of BZM and main impurity is increased from 1.71 to 5.83 with a purity reducing from 99.10% to 97.38%. Yet, a reduced linear least square regression slope is still greater than 0.9996. Although an optimal resolution is obtained with pH 6.50, pH 7.00 is still the choice to avoid reducing resolution of BZM and minor impurity and that of BZM and main forced degradation compound (around t_{R} = 3.016 min).
(7) For the solution stability test, a sum of BZM solution is put under a room temperature to be processed with HPLC analysis for three days, as shown in FIG.12. Consequently, the difference of BZM retention time is 0.64%; the resolution difference is 5.81%; the purity difference is -0.37%; the difference of a linear least square regression slope is -3.18%; and linear correlation coefficients are all grater than 0.9997. It shows that BZM dissolved in DMSO has a good stability and has little change in purity after reserving under a room temperature for three days.

Nevertheless, the present invention uses LC-MS/MS MRM to analyze quantities of the labeled ligand [¹²³I]IBZM) and the free ligand (BZM). Although [¹²⁷I]IBZM and [¹²³I]IBZM has different molecular weights (404 and 400), their chemical characteristics are similar, such as HPLC retention times, fragmentation pathways of parent molecules and working parameters for MRM transition. Hence, the present invention replaces reference material of [¹²³I]IBZM with nonradioactive IBZM (i.e. [¹²³I]IBZM) to obtain best conditions for HPLC and LC-MS/MS MRM. Then the best analytical parameters obtained for the nonradioactive IBZM are applied for analyzing results of LC-MS/MS MRM of [¹²³I]IBZM.

Quantities of the labeled ligand and the free ligand are analyzed through the following steps:
(a) An LC-MS/MS is obtained, which has an electrospray ionization (ESI) scan mode and a positive ion scan mode.
(b) Quantities of [¹²³I]IBZM and BZM are obtained through multiple reaction monitoring (MRM) by the LC-MS/MS.
(c) Fragmentation pathways of parent molecules of BZM, nonradioactive IBZM and radioactive IBZM ([¹²³I]IBZM) are obtained.
(d) A linearity for quantification of MRM transition of the BZM is obtained.
(e) A linearity for quantification of MRM transition of the nonradioactive IBZM is obtained.
(f) A linearity for quantification of MRM transition of the [¹²³I]IBZM is obtained.

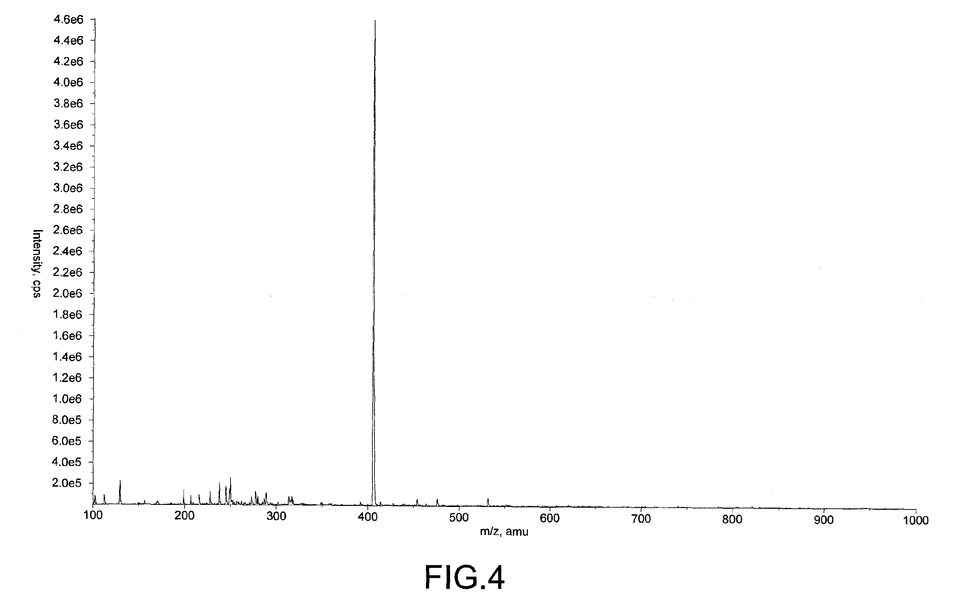

Firstly, a syringe pump is used to directly inject BZM or nonradioactive IBZM into the mass spectrometer at a flow rate of 10 µL/min for a Q1 scan, a precursor ion scan and a product ion scan. And, a declustering potential (DP), an entrance potential (EP), a collision energy (CE), a collision cell exit potential (CXP) are obtained. The best result is shown in FIG.4.

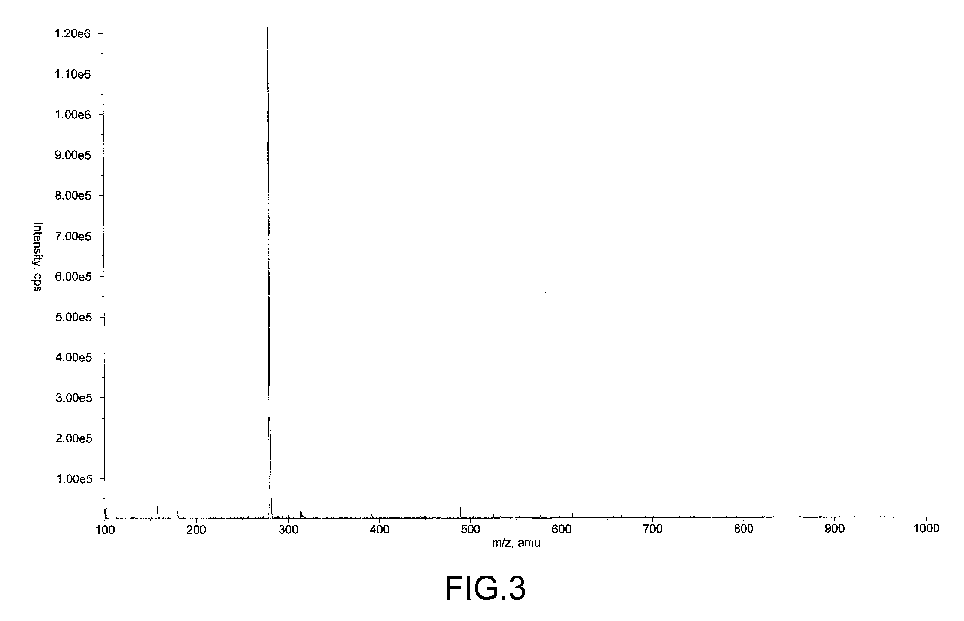

Through the Q1 scan, BZM (as shown in FIG.3) and nonradioactive IBZM (as shown in FIG.4) contain no obvious impurity. Through the precursor ion scan and the product ion scan, fragmentation ions of BZM are found to be m/z 151, m/z 136, m/z 129, m/z 1 1 2 and m/z 108; and those of nonradioactive IBZM are found to be m/z 276, m/z 262, m/z 234, m/z 150, m/z 129, m/z 112 and m/z 107. It shows that these two have very similar chemical structures.

For affirming the reproducibility of the above fragmentation ions and for affirming that the fragmentation ions can be applied in analyzing the quantification of the MRM transitions, HPLC is used as a sample introduction tool for obtaining linearity of BZM MRM transitions, where linearity of BZM MRM transitions include m/z 279→m/z 151, m/z 279→m/z 136, m/z 279→m/z 129, m/z 279→m/z 112, m/z 279→m/z 108; and linearity of nonradioactive IBZM MRM transitions include m/z 405→m/z 276, m/z 405→m/z 262, m/z 405→m/z 234, m/z 405→m/z 150, m/z 405→m/z 129, m/z 405→m/z 112, m/z 405→m/z 107.

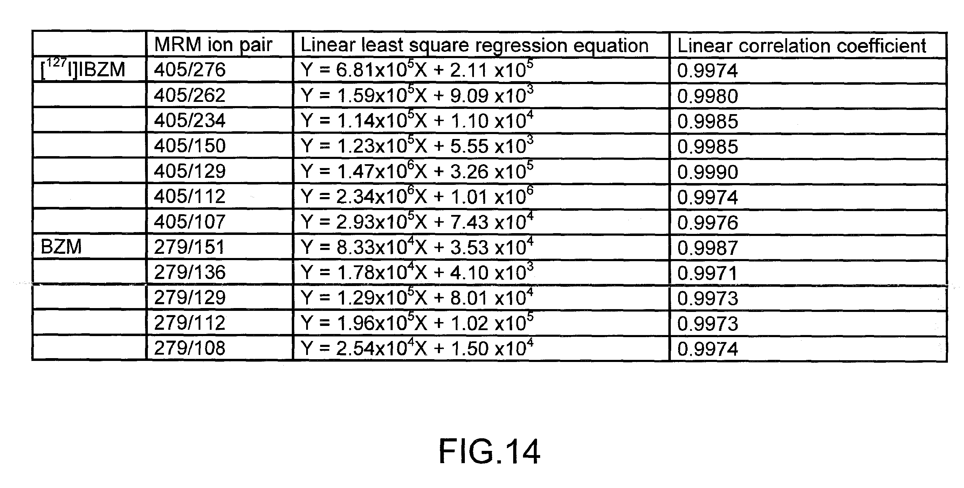

As shown in FIG.14, the linear least square regression of MRM transitions reveals that the five BZM MRM transitions and the seven nonradioactive IBZM MRM transition have good reproducibility and linearity; and their linear correlation coefficients are all greater than 0.9970. In addition, two most sensitive MRM precursors to product ion transitions of BZM and nonradioactive IBZM are the same, including m/z 129 and m/z 112.

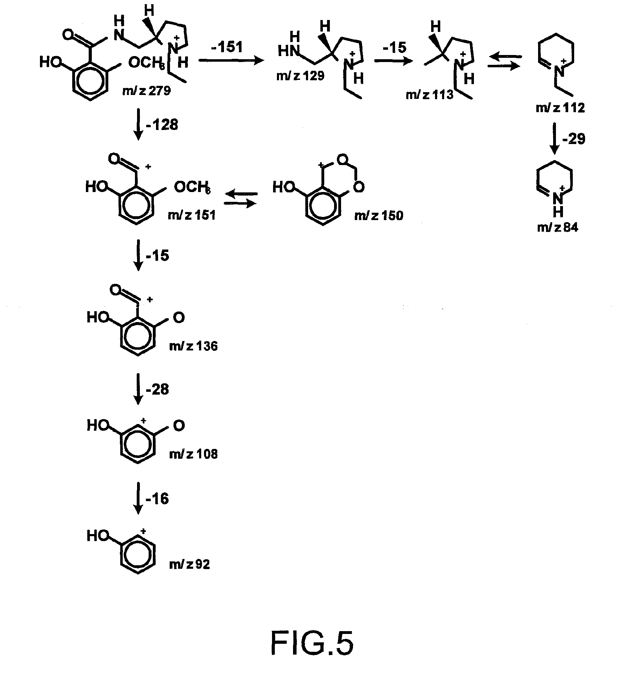

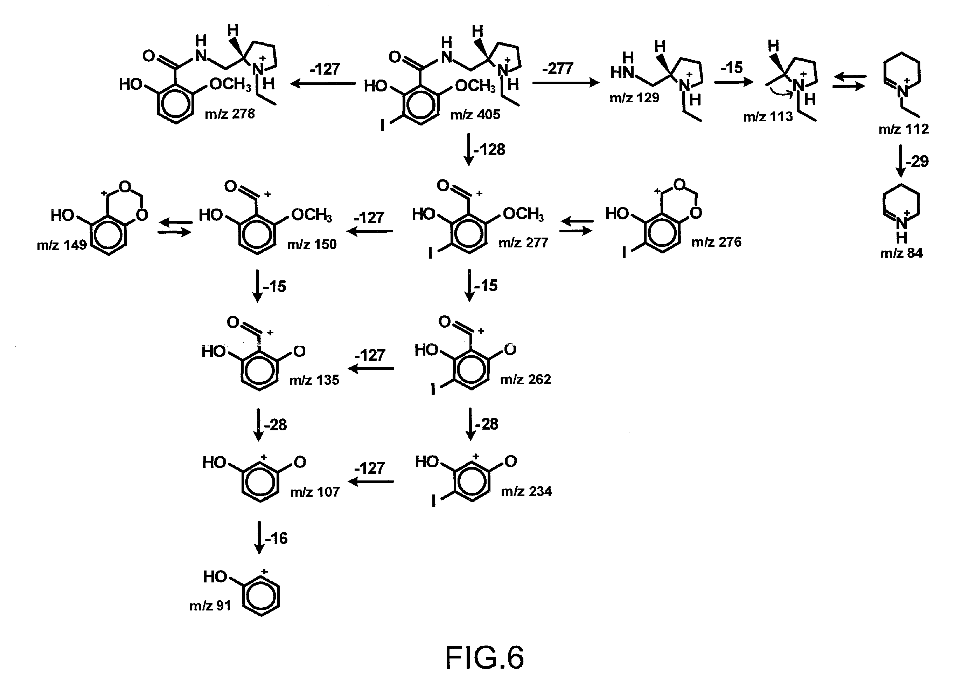

Through the precursor ion scan and the product ion scan of BZM and nonradioactive IBZM, fragmentation pathways of parent molecules of BZM and nonradioactive IBZM are obtained, as shown in FIG.5 and FIG.6. Therein, mass-to-charge ratios of most major product ion fragments can be found in chromatogram of the precursor ion scan and that of the product ion scan obtained through the mass spectrometer.

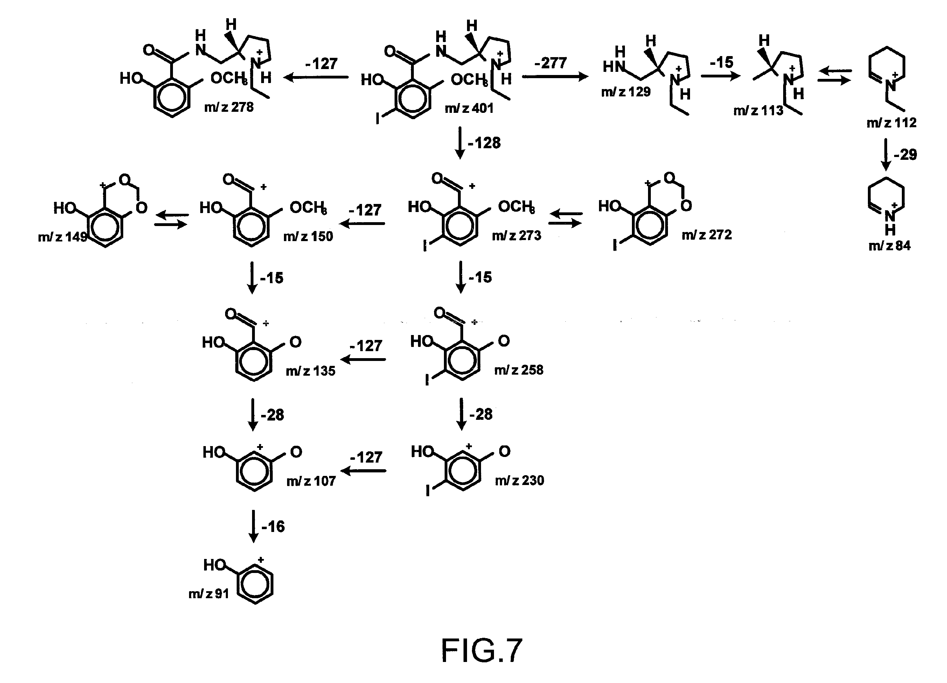

Because chemical behaviors of [¹²³I]IBZM and [¹²⁷I]IBZM are very similar, FIG.7 can be regarded as fragmentation pathways of [¹²³I]IBZM parent molecules. Hence, main MRM transitions of [¹²³I]IBZM has linearity of m/z 401→m/z 272, m/z 401→m/z 258, m/z 401→m/z 230, m/z 401→m/z 150, m/z 401→m/z 129, m/z 401→m/z 112, m/z 401→m/z 107. And. most sensitive MRM precursors to product ion transitions of BZM and nonradioactive IBZM are the same, including m/z 129 and m/z 112.

To sum up, the present invention is a method of obtaining a BZM purity, a quantity of [¹²³I]IBZM labeled ligand and a quantity of BZM free ligand, where HPLC is used to analyze a purity of BZM precursor of a striatal dopaminergic D2/D3 receptor SPECT imaging agent; and an LC-MS/MS is used to examine quantities of labeled ligand of [¹²³I]IBZM and free ligand of BZM in the striatal dopaminergic D2/D3 receptor SPECT imaging agent.

The preferred embodiment herein disclosed is not intended to unnecessarily limit the scope of the invention. Therefore, simple modifications or variations belonging to the equivalent of the scope of the clams and the instructions disclosed herein for a patent are all within the scope of the present invention.

## Claims

1. A method of obtaining a BZM purity, a quantity of [¹²³I]IBZM labeled ligand and a quantity of BZM free ligand, said BZM purity being obtained through steps of:
(a) obtaining a high performance liquid chromatography (HPLC) device; and
(b) examining [¹²³I]IBZM and BZM by said HPLC device to obtain specificity, linearity, accuracy, precision, limit of detection (LOD), limit of quantification (LOQ), robustness, and solution stability.

2. The method according to claim 1, wherein said quantity of [¹²³I]IBZM labeled ligand and said quantity of BZM free ligand being obtained through steps of:
(a) obtaining a liquid chromatograph tandem mass spectrometer (LC-MS/MS), said LC-MS/MS having an electrospray ionization (ESI) mode and a positive ion scan mode;
(b) obtaining a quantity of [¹²³I]IBZM and a quantity of BZM through multiple reaction monitoring (MRM) by said LC-MS/MS;
(c) obtaining parent molecule fragmentation pathways of BZM, nonradioactive IBZM and radioactive IBZM ([¹²³I]IBZM);
(d) obtaining a linearity of MRM transition of said BZM quantitatively;
(e) obtaining a linearity of MRM transition of said nonradioactive IBZM quantitatively; and
(f) obtaining a linearity of MRM transition of said [¹²³I]IBZM quantitatively.
